(19) Europäisches Patentamt
European Patent Office
Office européen des brevets

(11) EP 3 299 376 A1

(12) **EUROPEAN PATENT APPLICATION**
published in accordance with Art. 153(4) EPC

(43) Date of publication:
**28.03.2018 Bulletin 2018/13**

(21) Application number: **16796594.6**

(22) Date of filing: **20.05.2016**

(51) Int Cl.:
*C07F 9/655* (2006.01)
*A61K 8/67* (2006.01)
*A61K 31/665* (2006.01)
*A61P 17/00* (2006.01)
*A61Q 19/00* (2006.01)

(86) International application number:
**PCT/JP2016/065040**

(87) International publication number:
**WO 2016/186201 (24.11.2016 Gazette 2016/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.05.2015 JP 2015102878**

(71) Applicant: **Showa Denko K.K.**
**Tokyo 105-8518 (JP)**

(72) Inventors:
- **KATO, Eiko**
  **Tokyo 105-8518 (JP)**
- **ISHII, Hiroshi**
  **Tokyo 105-8518 (JP)**
- **SHIBUYA, Akira**
  **Tokyo 105-8518 (JP)**

(74) Representative: **Strehl Schübel-Hopf & Partner**
**Maximilianstrasse 54**
**80538 München (DE)**

# (54) TOCOPHEROL PHOSPHATE ESTER SALT AND METHOD FOR PRODUCING SAME, AND EXTERNAL SKIN PREPARATION

(57) A tocopherol phosphoric acid ester salt in which the tocopherol phosphoric acid ester salt is represented by Formula (1), and in the Formula (1), "R1", "R2", and "R3" each independently represents a hydrogen atom or a methyl group, "M" represents an alkali metal, and "a" is 1.10 or more and 2.00 or less.

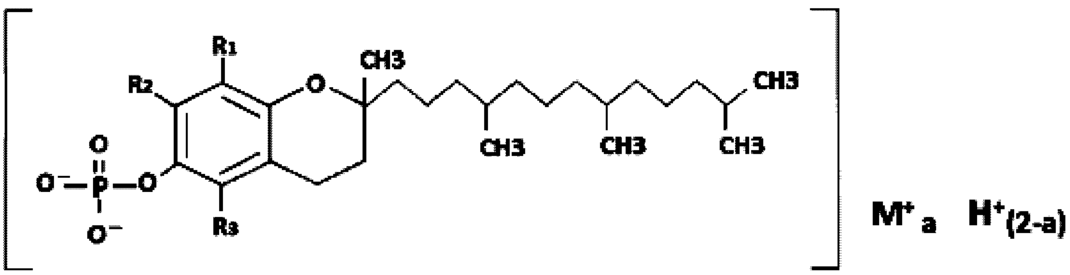

・・・(1)



Printed by Jouve, 75001 PARIS (FR)

## Description

### Technical Field

The present invention relates to a tocopherol phosphoric acid ester salt having excellent water solubility, a method for manufacturing the same, and a skin external preparation using the same.
Priority is claimed on Japanese Patent Application No. 2015-102878, filed on May 20, 2015, the content of which is incorporated herein by reference.

### Background Art

In the related art, it is known that tocopherols (a-tocopherol, β-tocopherol, γ-tocopherol, δ-tocopherol and the like), which are known as vitamin E, and derivatives thereof exhibit effects such as an antioxidant action, a biological membrane stabilization action, an immunopotentiation action, and a blood circulation promotion action. In order to take advantage of these effects, tocopherols and the derivatives thereof have conventionally been mixed into a skin external preparation.
As tocopherols and derivatives thereof which are mixed into a skin external preparation, a water-soluble tocopherol derivative such as a tocopherol phosphoric acid ester salt is known (for example, see PTL 1 to PTL 4).
PTL 1 proposes a whitening skin external preparation using a tocopherol phosphoric acid ester salt. PTL 2 proposes a skin external anti-wrinkle agent using a tocopherol phosphoric acid ester salt.

### Citation List

### Patent Literature

[PTL 1] Japanese Unexamined Patent Application, First Publication No. 2004-26817
[PTL 2] Japanese Unexamined Patent Application, First Publication No. 2008-7428
[PTL3] Japanese Unexamined Patent Application, First Publication No. S59-44375
[PTL 4] PCT International Publication No. WO 97/14705

### Summary of Invention

### Technical Problem

However, in the tocopherol phosphoric acid ester salt of the related art, water solubility is not sufficient. Therefore, there is a case where formulation is difficult at the time of manufacturing the skin external preparation including the tocopherol phosphoric acid ester salt of the related art.
The present invention is made in view of the circumstances described above, and has an object to provide a tocopherol phosphoric acid ester salt having excellent water solubility and a method for manufacturing the same. The present invention also has an object to provide a skin external preparation having excellent productivity by including a tocopherol phosphoric acid ester salt having excellent water solubility.

### Solution to Problem

In order to solve the problems described above, the present inventors diligently studied by focusing on a molar equivalent of an alkali metal with respect to 1 mole of a tocopherol phosphoric acid ester in the alkali metal salt of the tocopherol phosphoric acid ester. As a result, it was found that by making the molar equivalent of the alkali metal described above 1.10 or more, a tocopherol phosphoric acid ester salt having excellent water solubility can be obtained, and thus completed the present invention.
In the related art, only an alkali metal salt of the tocopherol phosphoric acid ester, of which the molar equivalent of the alkali metal is less than 1.10, was manufactured. As a result of the studies by the present inventors, the water solubility of the alkali metal salt of a tocopherol phosphoric acid ester of which the molar equivalent of the alkali metal is less than 1.10 is not sufficient.
The present invention adopts the following configurations.

<1> A tocopherol phosphoric acid ester salt, in which the tocopherol phosphoric acid ester salt is represented by

Formula (1).

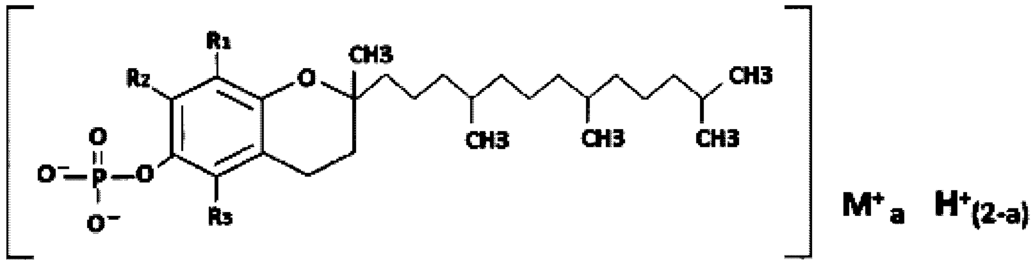

・・・(1)

(in the Formula (1), "R1", "R2", and "R3" each independently represents a hydrogen atom or a methyl group; "M" represents an alkali metal; and "a" is 1.10 or more and 2.00 or less).

<2> The tocopherol phosphoric acid ester salt according to <1>, in which in the Formula (1), the "a" is 1.10 or more and 1.70 or less.

<3> The tocopherol phosphoric acid ester salt according to <1> or <2>, in which in the Formula (1), "M" is a sodium.

<4> A skin external preparation including the tocopherol phosphoric acid ester salt according to any one of <1> to <3>.

<5> A method for manufacturing the tocopherol phosphoric acid ester salt according to any one of <1> to <3>, the method including: generating a tocopherol phosphoric acid ester by reacting a tocopherol and a phosphorylating agent, and then, dissolving a salt precipitated along with the tocopherol phosphoric acid ester by adding a sulfuric acid aqueous solution to a reaction solution including the tocopherol phosphoric acid ester; washing the reaction solution after dissolving the salt to remove a phosphoric acid therefrom; and neutralizing the tocopherol phosphoric acid ester with an alkali metal hydroxide to generate an alkali metal salt of the tocopherol phosphoric acid ester, in which an amount of the alkali metal hydroxide used in the neutralizing is controlled so that the number of moles of an alkali metal with respect to 1 mole of the tocopherol phosphoric acid ester is 1.17 to 1.88.

Advantageous Effects of Invention

In a tocopherol phosphoric acid ester salt of the present invention, a molar equivalent of an alkali metal with respect to 1 mole of a tocopherol phosphoric acid ester is 1.10 or more. Therefore, the tocopherol phosphoric acid ester salt has excellent water solubility.

A skin external preparation of the present invention includes the tocopherol phosphoric acid ester salt of the present invention. Therefore, formulation is easy, and productivity is excellent.

Description of Embodiments

Hereinafter, the present invention will be described in detail.

(Tocopherol Phosphoric Acid Ester Salt)

A tocopherol phosphoric acid ester salt of the present invention is represented by Formula (1) (in Formula (1), "R1", "R2", and "R3" each independently represents a hydrogen atom or a methyl group; "M" represents an alkali metal; and "a" is 1.10 or more and 2.00 or less).

Since the tocopherol phosphoric acid ester salt represented by Formula (1) has an asymmetric carbon atom at the second position of a chroman ring, there are d-isomer and 1-isomer, which are stereoisomers, and dl-isomer of the tocopherol phosphoric acid ester salt. The tocopherol phosphoric acid ester salt of the present invention includes any of these isomers.

It is preferable that the tocopherol phosphoric acid ester salt represented by Formula (1) be any of an α-tocopherol phosphoric acid ester salt in which all of "R1", "R2" and "R3" are methyl groups, a γ-tocopherol phosphoric acid ester salt in which "R1" and "R2" are methyl groups, and "R3" is a hydrogen atom, and a α-tocopherol phosphoric acid ester salt in which "R1" is a methyl group, and "R2" and "R3" are hydrogen atoms. Among these, particularly, the α-tocopherol phosphoric acid ester salt or the γ-tocopherol phosphoric acid ester salt is preferable since physiological activity is strong and it is easy to obtain.

A phosphoric acid group included in the tocopherol phosphoric acid ester salt represented by Formula (1) has a negative electric charge, and forms salts with one or more kinds of alkali metal ions having a positive electric charge.

The alkali metal "M" in Formula (1) is sodium, potassium or the like. The alkali metal "M" in Formula (1) may be one or more kinds. In particular, the alkali metal "M" is preferably sodium, since a tocopherol phosphoric acid ester salt having excellent water solubility is obtained. A sodium salt of the tocopherol phosphoric acid ester is preferable because it is a powder, and is easily handled. In a case where the tocopherol phosphoric acid ester salt represented by Formula (1) includes two or more kinds of the alkali metals "M", it is preferable that sodium be 90% or more.

In Formula (1), "a" means the number of moles (molar equivalent) of the alkali metal "M" with respect to 1 mole of the tocopherol phosphoric acid ester (represented by the chemical formula in parentheses in Formula (1)). In Formula (1), "a" is 1.10 or more and 2.00 or less, and is a numerical value obtained by Formula (2).

$$a = (\text{content of M} / \text{atomic weight of M}) / (\text{content of T} / \text{molecular weight of T}) \quad \cdots\cdot (2)$$

(in Formula (2), "M" represents an alkali metal, and "T" represents a tocopherol phosphoric acid ester; a content of "M" is a content (mass%) of the alkali metal in the tocopherol phosphoric acid ester salt; a content of "T" is a content (mass%) of the tocopherol phosphoric acid ester in the tocopherol phosphoric acid ester salt).

It is possible to obtain the content of the alkali metal "M" in Formula (2) by a method such as, for example, an ICP atomic emission spectrometry method, an atomic absorption spectrophotometry method, a flame emission spectrometry method, or ion chromatography.

It is possible to obtain the content of the tocopherol phosphoric acid ester T in Formula (2) by, for example, detecting with an ultraviolet-visible spectrometer using a column for high-performance liquid chromatography. As a column for high-performance liquid chromatography, for example, a column that is filled with a poly(meth)acrylate-based gel to which a long-chain alkyl group, preferably an octadecyl group, is bonded is used.

The water solubility of the tocopherol phosphoric acid ester salt represented by Formula (1) is influenced by a numerical value of "a" in Formula (1). If "a" in Formula (1) is less than 1.10, the water solubility of the tocopherol phosphoric acid ester salt is not sufficient. Therefore, there is a case where a problem occurs at the time of formulating a skin external preparation such as a skin toner. If "a" in Formula (1) is 1.10 or more, the water solubility of the tocopherol phosphoric acid ester salt is very favorable. The water solubility of the tocopherol phosphoric acid ester salt is improved as "a" in Formula (1) approaches 2.00. "a" in Formula (1) is preferably 1.15 or more, and more preferably 1.20 or more, in order to obtain a tocopherol phosphoric acid ester salt having excellent water solubility.

Specifically, in an aqueous solution of 1 mass% sodium salt of an $\alpha$-tocopherol phosphoric acid ester in which "a" in Formula (1) is 1.00, turbidity is visually observed. On the other hand, in an aqueous solution of 1 mass% sodium salt of an $\alpha$-tocopherol phosphoric acid ester in which "a" in Formula (1) is 1.10, turbidity is not observed. With respect to manufacturing the aqueous solution of the sodium salt of the $\alpha$-tocopherol phosphoric acid ester, a sodium salt of a $\alpha$-tocopherol phosphoric acid ester in which "a" in Formula (1) is 1.15 can be effectively dissolved in water, and the required time for dissolving it in water is one third the required time as compared to the case in which "a" is 1.10.

"a" in Formula (1) is preferably 2.00 or less, and more preferably 1.70 or less. If "a" in Formula (1) is 1.70 or less, a tocopherol phosphoric acid ester salt having sufficient oil solubility and high solubility in an organic solvent is formed. It is preferable that it be possible to easily and efficiently purify such a tocopherol phosphoric acid ester salt by crystallizing the tocopherol phosphoric acid ester salt using an organic solvent. If "a" in Formula (1) is 1.70 or less, the solubility in methyl-t-butyl ether (MTBE), which is an easily handled organic solvent, becomes particularly high.

If "a" in Formula (1) is 1.70 or less, in a case where the skin external preparation including the tocopherol phosphoric acid ester salt is manufactured, it is possible to prevent a salting-out effect due to the alkali metal "M" in the skin external preparation. Therefore, it is preferable that it be possible to manufacture a skin external preparation having excellent stability.

In order to obtain a tocopherol phosphoric acid ester salt of which the solubility in the organic solvent is high, and to prevent the salting-out effect due to the alkali metal "M" in the skin external preparation including the tocopherol phosphoric acid ester salt, "a" in Formula (1) is more preferably 1.60 or less, and further preferably 1.50 or less.

That is, if "a" is 1.10 or more and 1.70 or less, a balance between water solubility and oil solubility becomes excellent.

(Method for Manufacturing Tocopherol Phosphoric Acid Ester Salt)

It is possible to manufacture the tocopherol phosphoric acid ester salt of the embodiment, for example, using a manufacturing method in which processes of <1> to <4> described below are performed.

<1> A tocopherol phosphoric acid ester is generated by reacting a tocopherol and a phosphorylating agent. Thereafter, a sulfuric acid aqueous solution is added to a reaction solution including the tocopherol phosphoric acid ester, and a salt which is precipitated along with the tocopherol phosphoric acid ester is dissolved.

<2> A washing process for removing phosphoric acid ($H_3PO_4$) from the reaction solution is performed after dissolving the salt.

<3> An alkali metal salt of the tocopherol phosphoric acid ester is generated by neutralizing the tocopherol phosphoric acid ester with an alkali metal hydroxide (neutralization process).

<4> The alkali metal salt of the tocopherol phosphoric acid ester is purified as necessary.

In the process of generating the tocopherol phosphoric acid ester in <1>, it is possible to use $\alpha$-tocopherol, $\gamma$-tocopherol, $\sigma$-tocopherol, or the like as the tocopherol. The tocopherol may be any of these isomers.

As a phosphorylating agent, it is possible to use phosphorus oxychloride, trimetaphosphoric acid, polyphosphoric acid, or the like.

In <1>, in order to reliably react the tocopherol and the phosphorylating agent, it is preferable to use the phosphorylating agent to excess with respect to the tocopherol.

In the embodiment, the washing process for removing the phosphoric acid ($H_3PO_4$) in <2> is performed before neutralizing the tocopherol phosphoric acid ester. The phosphoric acid removed in the washing process is a by-product that is formed by reacting the excess phosphorylating agent not used in the reaction with the tocopherol and the sulfuric acid aqueous solution in the process of generating the tocopherol phosphoric acid ester in <1>. In the embodiment, even in a case where the phosphorylating agent is used to excess with respect to the tocopherol in <1>, the phosphoric acid is sufficiently removed by performing the washing process.

As an example of a washing process for removing the phosphoric acid included in the reaction solution, repeated washing is performed three or more times on an organic layer of the reaction solution including the tocopherol phosphoric acid ester, using water having twice or more mass of the organic layer.

In the process of neutralizing the tocopherol phosphoric acid ester in <3>, for example, it is possible to use a method of adding the alkali metal hydroxide dissolved in the solvent dropwise into the solution obtained by dissolving the tocopherol phosphoric acid ester in the solvent. As an alkali metal hydroxide, it is possible to use sodium hydroxide, potassium hydroxide, or the like.

In the embodiment, it is preferable that an amount of the alkali metal hydroxide used in the process of neutralizing the tocopherol phosphoric acid ester be adjusted so that the number of moles (molar equivalent) of the alkali metal with respect to 1 mole of the tocopherol phosphoric acid ester [(the number of moles of the alkali metal/the number of moles of the tocopherol phosphoric acid ester), also referred to as "b value", hereinafter] is 1.17 or more.

In this case, in the process of neutralizing the tocopherol phosphoric acid ester, a tocopherol phosphoric acid ester salt in which "a" in Formula (1) is 1.10 or more is easily obtained. The molar equivalent "b value" of the alkali metal is preferably 1.20 or more so that "a" in Formula (1) is 1.15 or more, and the molar equivalent "b value" of the alkali metal is preferably 1.30 or more so that "a" in Formula (1) is 1.20 or more.

The molar equivalent "b value" of the alkali metal is preferably 1.88 or less, and preferably 1.79 or less. If "b value" is 1.88 or less, a tocopherol phosphoric acid ester salt in which "a" in Formula (1) is 1.80 or less is obtained. If "b value" is 1.79 or less, a tocopherol phosphoric acid ester salt in which "a" in Formula (1) is 1.70 or less is obtained in the process of neutralizing the tocopherol phosphoric acid ester. The molar equivalent "b value" of the alkali metal is preferably 1.68 or less so that "a" in Formula (1) is 1.60 or less, and the molar equivalent "b value" of the alkali metal is preferably 1.58 or less so that "a" in Formula (1) is 1.50 or less.

In the embodiment, in a case where the process of purifying the alkali metal salt of the tocopherol phosphoric acid ester in <4> is performed, it is preferable to purify the alkali metal salt of the tocopherol phosphoric acid ester by crystallizing the alkali metal salt of the tocopherol phosphoric acid ester using an organic solvent. In this case, as an organic solvent, it is possible to use methanol, acetone, methyl-t-butyl ether (MTBE), or the like. As an organic solvent, it is preferable to use methyl-t-butyl ether since it is easily handled.

In the manufacturing method of the embodiment, before the neutralization process <3> of neutralizing the tocopherol phosphoric acid ester with the alkali metal hydroxide is performed, the washing process <2> for removing the phosphoric acid ($H_3PO_4$) as the by-product is performed. Therefore, in the neutralization process <3>, the alkali metal hydroxide is prevented from being consumed by reacting with the phosphoric acid as the by-product. Accordingly, "b value", which is the number of moles (molar equivalent) of the alkali metal used in the neutralization process with respect to 1 mole of the tocopherol phosphoric acid ester, approximates to "a" in Formula (1) as the number of moles (molar equivalent) of the alkali metal "M" with respect to 1 mole of the tocopherol phosphoric acid ester in the alkali metal salt generated after the neutralization. Therefore, by adjusting the amount of the alkali metal hydroxide used in the neutralization process, a tocopherol phosphoric acid ester salt in which "a" in Formula (1) is 1.10 or more is easily and reliably obtained.

On the contrary, in the method for manufacturing the tocopherol phosphoric acid ester salt in the related art, a difference between the molar equivalent of the alkali metal used in the neutralization process and the molar equivalent

of "a" in Formula (1) in the alkali metal salt generated after the neutralization was large. Therefore, before performing the neutralization process, it was difficult to predict "a" in Formula (1) in the alkali metal salt generated after the neutralization. For example, in a case where the molar equivalent of the alkali metal used in the neutralization process was 1.2, "a" in Formula (1) in the alkali metal salt generated after the neutralization was approximately 1.0.

As a result of diligent studies, the present inventors found that the alkali metal hydroxide was consumed without contributing to the neutralization of the tocopherol phosphoric acid ester, since the phosphoric acid ($H_3PO_4$) as the by-product formed in the process of generating the tocopherol phosphoric acid ester reacted with the alkali metal hydroxide used in the neutralization process. It was also found that 10% to 40% of the molar equivalent of the alkali metal used in the neutralization process was consumed by the reaction with the phosphoric acid as the by-product. Accordingly, it is estimated that "a" in Formula (1) in the alkali metal salt generated after the neutralization is smaller than the molar equivalent of the alkali metal used in the neutralization process.

In the tocopherol phosphoric acid ester salt of the embodiment, "a" in Formula (1),which is the molar equivalent of the alkali metal with respect to 1 mole of the tocopherol phosphoric acid ester, is 1.10 or more. Therefore, the tocopherol phosphoric acid ester salt of the embodiment has excellent water solubility. Accordingly, in a case of manufacturing the skin external preparation including the same, it is possible to easily perform formulation.

In a case where "a" in Formula (1) is 1.70 or less, the tocopherol phosphoric acid ester salt of the embodiment has sufficient oil solubility, and has excellent water solubility. Accordingly, it is possible to easily manufacture various forms of skin external preparations such as a cream or a skin toner including the same. In the tocopherol phosphoric acid ester salt of the embodiment, in a case where "a" in Formula (1) is 1.70 or less, the salting-out effect due to the alkali metal "M" in the skin external preparation including the same is prevented. Therefore, a skin external preparation having excellent stability is obtained.

In the tocopherol phosphoric acid ester salt of the embodiment, in a case where "a" in Formula (1) is 1.70 or less, it is possible to reduce the amount of a neutralizing agent used when formulating the skin external preparation including the same. Therefore, it is possible to prevent an inorganic salt, increased by the neutralizing agent added when formulating the skin external preparation, from precipitating in the skin external preparation and generating a rough texture.

Since it is possible to manufacture a tocopherol phosphoric acid ester salt of the embodiment as a solid powder, effects such as ease of transport, excellent preservation stability, or ease of mixing into a skin external preparation, are obtained.

When in contact with the skin, the tocopherol phosphoric acid ester salt of the embodiment becomes tocopherol by breaking an ester bond after permeating into the skin, and a collagen synthesis promotion effect and a collagen decomposition inhibiting effect are exhibited. Therefore, by bringing the skin external preparation including the tocopherol phosphoric acid ester salt of the embodiment into contact with the skin, it is possible to expect an effect of preventing and improving morphological change of the skin due to aging. Accordingly, it is possible to widely use the tocopherol phosphoric acid ester salt of the embodiment in various kinds of skin external preparations to achieve a skin-beautifying effect.

(Skin External Preparation)

Next, the skin external preparation of the present invention will be described.

The skin external preparation of the embodiment includes the tocopherol phosphoric acid ester salt represented by Formula (1).

It is preferable that the skin external preparation of the embodiment includes the tocopherol phosphoric acid ester salt at 0.01 to 20 mass%. If the content of the tocopherol phosphoric acid ester salt is 0.01 mass% or more, the beneficial effect caused by including the tocopherol phosphoric acid ester salt is easily exhibited. In order to obtain the beneficial effect caused by including the tocopherol phosphoric acid ester salt, it is preferable to include the tocopherol phosphoric acid ester salt at 0.03 mass% or more, and it is more preferable to include the tocopherol phosphoric acid ester salt at 0.05 mass% or more.

If the content of the tocopherol phosphoric acid ester salt is 20 mass% or less, it is easy to dissolve and/or disperse the tocopherol phosphoric acid ester salt uniformly in the skin external preparation, and a skin external preparation with ease of formulation and excellent productivity can be obtained. Moreover, if the content of the tocopherol phosphoric acid ester salt is 20 mass% or less, it is possible to prevent the salting-out effect due to the alkali metal "M" in the skin external preparation. Therefore, a skin external preparation having excellent stability is produced. In order to uniformly dissolve and/or disperse the tocopherol phosphoric acid ester salt and improve the stability, it is more preferable to include the tocopherol phosphoric acid ester salt at 10 mass% or less, and it is further preferable to include the tocopherol phosphoric acid ester salt at 5 mass% or less.

In the skin external preparation of the embodiment, it is possible to include other ingredients such as those generally used for a skin external preparation, in addition to the tocopherol phosphoric acid ester salt. As other ingredients which may be mixed into the skin external preparation of the embodiment, for example, an ascorbic acid derivative,

hydrocarbons, natural fats and oils, fatty acids, higher alcohols, alkyl glyceryl ethers, esters, silicone oils, macromolecules, lower alcohols, polyhydric alcohols, surfactants, ultraviolet-absorbing agents, powders and coloring materials, plant extracts, amino acids and peptides, vitamins and vitamin-like active factors, antiseptic agents, antioxidants, sequestering agents, moisturizing agents, anti-inflammatory agents, pH-regulating agents, salts, α-hydroxy acids, whitening agents, essential oils, terpenes, perfumes, water, and the like can be used.

As an ascorbic acid derivative, for example, ascorbic acid-2-phosphoric acid, ascorbic acid-2-glucoside, ascorbic acid-6-palmitic acid, ascorbic acid-2-phosphoric acid-6-palmitic acid, ascorbic acid-2-phosphoric acid-6-hexyldecanoic acid, ascorbic acid-6-tetraisopalmitic acid, or the like can be used. As an ascorbic acid derivative salt, an alkali metal salt or an alkaline earth metal salt of the compound described above can be used.

As hydrocarbons, for example, squalene, mineral oil, and the like can be used.

As natural fats and oils, for example, jojoba oil, olive oil, palm oil, camellia oil, shea butter, and the like can be used.

As fatty acids, for example, lauric acid, myristic acid, palmitic acid, stearic acid, behenic acid, oleic acid, isostearic acid, 12-hydroxystearic acid, undecylenic acid, coconut oil fatty acid, and the like can be used.

As higher alcohols, for example, isostearyl alcohol, cetanol, stearyl alcohol, behenyl alcohol, cetostearyl alcohol, and the like can be used.

As alkyl glyceryl ethers, for example, batyl alcohol, chimyl alcohol, selachyl alcohol, isostearyl glyceryl ether, and the like can be used.

As esters, for example, isopropyl palmitate, octyl dodecyl myristate, isononyl isononanoate, and the like can be used.

As silicone oils, for example, methylpolysiloxane, alkyl-modified silicone, and the like are used.

As macromolecules, for example, xanthan gum, hydroxyethyl cellulose, sodium polyacrylate, carboxyvinyl polymer, and the like can be used.

As lower alcohols, for example, ethanol, isopropyl alcohol, 1-butanol, 2-butanol, benzyl alcohol, and the like can be used.

As polyhydric alcohols, for example, propylene glycol, dipropylene glycol, glycerin, 1,3-butanediol, 1,2-pentanediol, 1,2-hexanediol, and the like can be used.

As a surfactant, for example, an anionic surfactant, a cationic surfactant, an amphoteric surfactant, a nonionic surfactant, or a natural surfactant can be used.

As an anionic surfactant, for example, sodium stearate, sodium lauryl sulfate, triethanolamine lauryl sulfate, sodium cetyl sulfate, sodium polyoxyethylene lauryl ether sulfate, or the like can be used.

As a cationic surfactant, for example, lauryl trimethyl ammonium chloride, cetyl trimethyl ammonium chloride, or the like can be used.

As an amphoteric surfactant, for example, sodium lauryl aminopropionate, lauryl dimethylaminoacetic acid betaine, or the like can be used.

As a nonionic surfactant, for example, polyoxyethylene alkyl ether, polyoxyethylene hardened castor oil, sucrose fatty acid ester, or the like can be used.

As a natural surfactant, for example, hydrogenated soybean phospholipid, phosphatidyl serine, sodium deoxycholate, sophorolipid, or the like can be used.

As an ultraviolet-absorbing agent, for example, a paraaminobenzoic acid derivative, a cinnamic acid derivative, a urocanic acid derivative, a benzophenone derivative, or the like can be used.

As powders and coloring materials, for example, zinc oxide, titanium oxide, and the like can be used.

As amino acids and peptides, for example, collagen, wheat peptide, and the like can be used.

As vitamins and vitamin-like active factors, for example, vitamins A, carotenoids, vitamins B2, vitamins D, oil-soluble vitamins E, ubiquinones, vitamins K, carnitine, ferulic acid, y-orizanol, α-lipoic acid, orotic acid, and the like can be used.

As an antiseptic agent, for example, butyl parahydroxybenzoic acid, propyl parahydroxybenzoic acid, methyl parahydroxybenzoic acid, phenoxyethanol, or the like can be used.

As an antioxidant, for example, butylhydroxyanisole, butylhydroxytoluene, propyl gallate, erythorbic acid, sodium erythorbate, parahydroxyanisole, octyl gallate, or the like can be used.

As a sequestering agent, for example, edetic acid, sodium citrate, or the like can be used.

As a moisturizing agent, for example, hyaluronic acid, sodium hyaluronate, sodium chondroitin sulfate, sodium lactate, sodium pyrrolidone carboxylate, betaine, a lactic acid bacteria culture solution, yeast extract, ceramide, or the like can be used.

As an anti-inflammatory agent, for example, glycyrrhizic acid, trisodium glycyrrhizinate, dipotassium glycyrrhizinate, monoammonium glycyrrhizinate, β-glycyrrhetinic acid, glyceryl glycyrrhetinate, stearyl glycyrrhetinate, lysozyme chloride, hydrocortisone, allantoin, or the like can be used.

As a pH-regulating agent, for example, sodium hydroxide, potassium hydroxide, triethanolamine, or the like can be used.

As salts, for example, sodium chloride, potassium chloride, magnesium chloride, sodium sulfate, and the like can be used.

As α-hydroxy acids, for example, citric acid, glycolic acid, tartaric acid, lactic acid, and the like can be used.

As a whitening agent, for example, arbutin, α-arbutin, a placenta extract, or the like can be used.

As terpenes, for example, pinene, terpinene, terpinolene, myrcene, longifilene, and the like can be used.

The skin external preparation of the embodiment may further include an existing cosmetic raw material, as necessary, in addition to the ingredients described above.

As a cosmetic raw material, for example, a cosmetic raw material that is described in Cosmetic raw material standards second edition annotation edited by Pharmaceutical and Medical Device Regulatory Science Society of Japan, 1984 (Yakuji Nippo Ltd. Company), Cosmetic raw material nonstandard ingredient standards supervised by Evaluation and Registration Division of Pharmaceutical Affairs Bureau in Ministry of Health and Welfare, 1993 (Yakuji Nippo Ltd. Company), Cosmetic raw material nonstandard ingredient standards addendum supervised by Evaluation and Registration Division of Pharmaceutical Affairs Bureau in Ministry of Health and Welfare, 1993 (Yakuji Nippo Ltd. Company), Cosmetics assortment licensing standards supervised by Evaluation and Registration Division of Pharmaceutical Affairs Bureau in Ministry of Health and Welfare, 1993 (Yakuji Nippo Ltd. Company), Cosmetic assortment mixing ingredient standards supervised by Evaluation and Registration Division of Pharmaceutical Affairs Bureau in Ministry of Health and Welfare, 1997 (Yakuji Nippo Ltd. Company), Cosmetic ingredient dictionary, Heisei 3 (Nikko Chemicals Co., Ltd.) and New cosmetic functional material 300, 2002 (CMC Publishing), or the like can be used.

The skin external preparation of the embodiment may be in any dosage form or form as long as it can be used in contact with the skin at the time of use.

It is preferable that the skin external preparation of the present embodiment be in a dosage form or form which is suitable for applying to a part of the skin where the effects of including the tocopherol phosphoric acid ester salt are needed.

As a form of the skin external preparation, for example, a skin milk, a skin cream, a foundation cream, a massage cream, a cleansing cream, a shaving cream, a cleansing foam, a skin toner, a lotion, a pack, a lipstick, a blusher, an eye shadow, a manicure, a soap, a body shampoo, a hand soap, a shampoo, a rinse, a hair tonic, a treatment, a hair cream, a hair spray, a hair growth agent, a hair oil, a hair dye, a hairdressing charge, a depilatory, an anti-dandruff agent, a toothpaste, a denture adhesive, a gargle, a permanent wave agent, a curling agent, a styling agent, an ointment, a cataplasm, a tape, a bathwater additive, an antiperspirant, an anti-sunburn agent, or the like can be used.

As a dosage form of the skin external preparation, any of solid, liquid, semi-solid and gas may be used. Specifically, a dosage form such as powder, granules, tablet form, gel form or foam form can be used.

The skin external preparation of the embodiment is used regardless of the sex or age of a user. Moreover, the skin external preparation of the embodiment may be used in contact with the skin of animals.

It is possible to manufacture the skin external preparation of the embodiment using the ingredient including the tocopherol phosphoric acid ester salt of the embodiment at a predetermined content, and dissolving, mixing, or dispersing it in accordance with a normal method, depending on the dosage form or form thereof.

The skin external preparation of the embodiment includes the tocopherol phosphoric acid ester salt of the embodiment having excellent water solubility described above. Accordingly, the skin external preparation of the embodiment has excellent productivity since the formulation thereof is easy.

[Examples]

Hereinafter, the present invention will be specifically described with reference to Examples, but the present invention is not limited in any way by Examples.

In Examples and Comparative Examples, "%" means mass%, and unless stated to the contrary, a total amount of all ingredients is 100 mass%. "a value" and "b value" were calculated by the method described later, and the values thereof are shown in Table 1.

(Example 1)

A solution was prepared by dissolving 25.0 g (0.05 mole) of dl-α-tocopherol in 75 ml of toluene including 9.3 g of pyridine. The prepared solution was cooled in an ice bath to 0°C, and 9.8 g (0.064 mole) of phosphorus oxychloride as a phosphorylating agent was added dropwise for 5 minutes while stirring the solution. After completion of the dropwise addition, reaction was performed at room temperature for 3 hours, and a tocopherol phosphoric acid ester was generated. Next, 50 ml of a 6N-sulfuric acid aqueous solution was added to the solution including the tocopherol phosphoric acid ester, and the solution was heated under reflux for 3 hours.

Thereafter, the solution including the tocopherol phosphoric acid ester was put into a separating funnel, and was separated into an organic layer including the tocopherol phosphoric acid ester and a water layer. The separated

organic layer was washed with a IN-hydrochloric acid aqueous solution. Thereafter, in order to remove phosphoric acid included in the organic layer, the organic layer was washed three times using water having twice the mass of the organic layer.

The organic layer after washing was concentrated and dried with an evaporator. Thereafter, 100 ml of 1-propanol was added to dissolve the dried material, 25 ml of methanol in which 2.4 g (0.059 mole) of sodium hydroxide was dissolved was added dropwise, neutralization was performed by warming to 35°C to 40°C for 1 hour, and a precipitate was filtrated.

Thereafter, the filtered precipitate was dissolved in 1 liter of methanol, and was concentrated to 150 ml, thereby, a concentrated solution was prepared. Next, 20 ml of acetone was added dropwise to the concentrated solution, and a white precipitate was precipitated. Thereafter, the precipitate was washed with acetone, and was dried under reduced pressure, thereby, 19.1 g of the sodium salt of the tocopherol phosphoric acid ester was obtained as a white powder.

(Example 2)

13.7 g (0.09 mole) of phosphorus oxychloride as the phosphorylating agent was dissolved in 40 g of methyl-t-butyl ether (MTBE). 30.0 g (0.06 mole) of dl-α-tocopherol was dissolved in 36 g of methyl-t-butyl ether (MTBE) including 11.2 g of pyridine.

Thereafter, a methyl-t-butyl ether solution including dl-α-tocopherol was added dropwise from a funnel to a methyl-t-butyl ether solution including phosphorus oxychloride while maintaining a solution temperature of 50°C or lower, and the reaction was performed by stirring for 30 minutes, thereby, a tocopherol phosphoric acid ester was generated. Next, 97 g of a 15% sulfuric acid aqueous solution was added to the solution including the tocopherol phosphoric acid ester while maintaining the solution temperature of 40°C or lower, and stirring was performed for 30 minutes.

Thereafter, in the same manner as Example 1, the solution including the tocopherol phosphoric acid ester was separated into the organic layer and the water layer. Thereafter, in the same manner as Example 1, except that 25 ml of methanol in which 2.9 g (0.072 mole) of sodium hydroxide was dissolved was used, 23.4 g of the sodium salt of the tocopherol phosphoric acid ester was obtained as a white powder.

(Example 3)

In the same manner as Example 2, except that 25 ml of methanol in which 3.1 g (0.078 mole) of sodium hydroxide was dissolved was used, the sodium salt of the tocopherol phosphoric acid ester was obtained as a white powder.

(Example 4)

In the same manner as Example 2, except that 25 ml of methanol in which 3.3 g (0.083 mole) of sodium hydroxide was dissolved was used, the sodium salt of the tocopherol phosphoric acid ester was obtained as a white powder.

(Comparative Example 1)

In the same manner as Example 1, the process was performed until the solution including the tocopherol phosphoric acid ester was separated into the organic layer including the tocopherol phosphoric acid ester, and the water layer. Next, the separated organic layer was washed with a IN-hydrochloric acid aqueous solution, and was dried with anhydrous sodium sulfate, without performing washing to remove the phosphoric acid included in the organic layer.

Thereafter, the organic layer which was dried with anhydrous sodium sulfate was concentrated and dried with an evaporator, and the same process as Example 1 was performed. Thereby, 18.9 g of the sodium salt of the tocopherol phosphoric acid ester was obtained as a white powder.

(Comparative Example 2)

In the same manner as Example 2, the process was performed until the solution including the tocopherol phosphoric acid ester was separated into the organic layer including the tocopherol phosphoric acid ester, and the water layer. Next, the separated organic layer was washed with a IN-hydrochloric acid aqueous solution. Thereafter, the same process as Example 2 was performed, except that the organic layer was washed two times using water having the same mass as that of the organic layer, in order to remove the phosphoric acid included in the organic layer. Thereby, 23.2 g of the sodium salt of the tocopherol phosphoric acid ester was obtained as a white powder.

(Comparative Example 3)

In the same manner as Example 2, the process was performed until the solution including the tocopherol phosphoric acid ester was separated into the organic layer including the tocopherol phosphoric acid ester, and the water layer. Next, the separated organic layer was washed with the IN-hydrochloric acid aqueous solution. Thereafter, the same process as Example 2 was performed, except that the organic layer was washed two times using water having twice the mass of the organic layer, in order to remove the phosphoric acid included in the organic layer. Thereby, 23.1 g of the sodium salt of the tocopherol phosphoric acid ester was obtained as a white powder.

(Comparative Example 4)

In the same manner as Example 3, the process was performed until the solution including the tocopherol phosphoric acid ester was separated into the organic layer including the tocopherol phosphoric acid ester, and the water layer. Next, the separated organic layer was washed with a IN-hydrochloric acid aqueous solution. Thereafter, the same process as Example 3 was performed, except that the separated organic layer was dried with anhydrous sodium sulfate, without performing washing to remove the phosphoric acid included in the organic layer. Thereby, the sodium salt of the tocopherol phosphoric acid ester was obtained as a white powder.

(Comparative Example 5)

In the same manner as in Example 4, the process was performed until the solution including the tocopherol phosphoric acid ester was separated into the organic layer including the tocopherol phosphoric acid ester, and the water layer. Next, the separated organic layer was washed with the IN-hydrochloric acid aqueous solution. Thereafter, the same process as Example 4 was performed, except that the separated organic layer was dried with anhydrous sodium sulfate, without performing washing to remove the phosphoric acid included in the organic layer. Thereby, the sodium salt of the tocopherol phosphoric acid ester was obtained as a white powder.

(Example 5)

In the same manner as Example 2, except that 25 ml of methanol in which 4.1 g (0.103 mole) of sodium hydroxide was dissolved was used, the sodium salt of the tocopherol phosphoric acid ester was obtained as a white powder.

(Example 6)

In the same manner as Example 2, except that 25 ml of methanol in which 4.5 g (0.112 mole) of sodium hydroxide was dissolved was used, the sodium salt of the tocopherol phosphoric acid ester was obtained as a white powder.

(Example 7)

In the same manner as Example 1, except that 3.3 g (0.059 mole) of potassium hydroxide was used instead of sodium hydroxide, the potassium salt of the tocopherol phosphoric acid ester was obtained as a white powder.

(Comparative Example 6)

In the same manner as Comparative Example 1, except that 3.3 g (0.059 mole) of potassium hydroxide was used instead of sodium hydroxide, the potassium salt of the tocopherol phosphoric acid ester was obtained as a white powder.

(Calculation of "a Value")

Regarding the sodium salts (or potassium salts) of the tocopherol phosphoric acid esters obtained in Example 1 to Example 7, and Comparative Example 1 to Comparative Example 6, the molar equivalent (which may be referred to as "a value", hereinafter) of the alkali metal "M" with respect to 1 mole of the tocopherol phosphoric acid ester was calculated using Formula (2). The calculated "a values" are illustrated in Table 1 to Table 3.

The content of the alkali metal "M" in Formula (2) was obtained by a method described below using atomic absorption spectrometry (flame method).

An aqueous solution of approximately 10 ppm (mass standard) of the sodium salt (or potassium salt) of the tocopherol phosphoric acid ester each obtained in Example 1 to Example 7, and Comparative Example 1 to Comparative Example 6, was prepared, and a sample solution was formed. Then, a concentration (ppm) of sodium (or potassium) in the sample solution was calculated using a calibration curve obtained from absorbance of a standard solution under the following conditions. The content (mass%) of the alkali metal "M" in the sodium salt (or potassium salt) of each tocopherol phosphoric acid ester was obtained by the following formula using the same.
Content (mass%) of sodium (or potassium) in sodium salt (or potassium salt) of tocopherol phosphoric acid ester = concentration (ppm) of sodium (or potassium) in sample solution × dilution rate of sample solution × 10000

<Absorbance Measurement Conditions of Sodium in Sample Solution>

Combustible gas: acetylene
Combustion supporting gas: air
Lamp: sodium hollow cathode lamp
Wavelength: 589.0 nm

<Absorbance Measurement Conditions of Potassium in Sample Solution>

Combustible gas: acetylene
Combustion supporting gas: air
Lamp: potassium hollow cathode lamp
Wavelength: 766.5 nm

The content (mass%) of a tocopherol phosphoric acid ester T in Formula (2) was calculated using high-performance liquid chromatography (HPLC), preparing the calibration curve using a reference standard, under the measurement conditions described below, and analyzing the sample solution (of which the concentration of the tocopherol phosphoric acid ester was approximately 1000 ppm (mass standard)) using the same.

<Measurement Conditions>

Column: Asahipak ODP-50 6D (Shodex (registered trademark))
Eluent: solution in which sodium acetate was dissolved in 1% hydrous methanol to have the concentration of 0.1 mol/L
Flow rate: 0.5 ml/minute
Column temperature: 40°C
Detector: UV 287 nm

(Calculation of "b Value")

In Example 1 to Example 7, and Comparative Example 1 to Comparative Example 6, the number of moles (molar equivalent) of the sodium hydroxide (or potassium hydroxide) used in the process of neutralizing the tocopherol phosphoric acid ester with respect to 1 mole of the tocopherol phosphoric acid ester [(the number of moles of the sodium hydroxide (or potassium hydroxide)/the number of moles of the tocopherol phosphoric acid ester), also referred to as "b value", hereinafter] was calculated. The calculation was made by assuming that the number of moles of the tocopherol phosphoric acid ester was the number of moles of dl-α-tocopherol used as raw material. The results thereof are illustrated in Table 1 to Table 3.
The number of moles of the sodium hydroxide (or potassium hydroxide) and dl-α-tocopherol used in generating the sodium salt (or potassium salt) of the tocopherol phosphoric acid ester are illustrated in Table 1 to Table 3.

(Water Solubility)

The water solubility of the sodium salt (or potassium salt) of the tocopherol phosphoric acid ester obtained in Example 1 to Example 7, and Comparative Example 1 to Comparative Example 6 was evaluated by the method described

below.

1.0 g of the sodium salt (or potassium salt) of the tocopherol phosphoric acid ester was put into 100 g of water at room temperature (25°C), was stirred, and was dissolved. Then, the state 3 hours from dissolving was visually observed. The results thereof are illustrated in Table 1 to Table 3.

(Solvent Solubility)

The solubility of the sodium salt of the tocopherol phosphoric acid ester obtained in Example 5 and Example 6 in methyl-t-butyl ether (MTBE) was evaluated by the method described below.

1.0 g of the sodium salt of the tocopherol phosphoric acid ester was put into 100 g of 2% hydrous MTBE at room temperature (25°C), was stirred, and was dissolved, and the state thereof was visually observed. The results thereof are illustrated in Table 2.

[Table 1]

| | RAW MATERIAL | | | | | |
|---|---|---|---|---|---|---|
| | NUMBER OF MOLES OF SODIUM HYDROXIDE (MOL) | NUMBER OF MOLES OF TOCOPHEROL (MOL) | b VALUE | a VALUE | b VALUE - a VALUE | WATER SOLUBILITY |
| EXAMPLE 1 | 0.059 | 0.05 | 1.18 | 1.12 | 0.06 | TRANSPARENCY |
| EXAMPLE 2 | 0.072 | 0.06 | 1.20 | 1.15 | 0.05 | TRANSPARENCY |
| EXAMPLE 3 | 0.078 | 0.06 | 1.30 | 1.24 | 0.06 | TRANSPARENCY |
| EXAMPLE 4 | 0.083 | 0.06 | 1.38 | 1.32 | 0.06 | TRANSPARENCY |
| COMPARATIVE EXAMPLE 1 | 0.059 | 0.05 | 1.18 | 0.94 | 0.24 | WHITE TURBIDITY |
| COMPARATIVE EXAMPLE 2 | 0.072 | 0.06 | 1.20 | 1.00 | 0.20 | WHITE TURBIDITY |
| COMPARATIVE EXAMPLE 3 | 0.072 | 0.06 | 1.20 | 1.05 | 0.15 | WHITE TURBIDITY |
| COMPARATIVE EXAMPLE 4 | 0.078 | 0.06 | 1.30 | 1.00 | 0.30 | WHITE TURBIDITY |
| COMPARATIVE EXAMPLE 5 | 0.083 | 0.06 | 1.38 | 1.05 | 0.33 | WHITE TURBIDITY |

[Table 2]

| | RAW MATERIAL | | | | | | |
|---|---|---|---|---|---|---|---|
| | NUMBER OF MOLES OF SODIUM HYDROXIDE (MOL) | NUMBER OF MOLES OF TOCOPHEROL (MOL) | b VALUE | a VALUE | b VALUE - a VALUE | WATER SOLUBILITY | MTBE SOLUBILITY |
| EXAMPLE 5 | 0.103 | 0.06 | 1.72 | 1.64 | 0.08 | TRANSPARENCY | TRANSPARENCY |
| EXAMPLE 6 | 0.112 | 0.06 | 1.87 | 1.78 | 0.09 | TRANSPARENCY | WHITE TURBIDITY |

[Table 3]

| | RAW MATERIAL | | | | | |
|---|---|---|---|---|---|---|
| | NUMBER OF MOLES OF POTASSIUM HYDROXIDE (MOL) | NUMBER OF MOLES OF TOCOPHEROL (MOL) | b VALUE | a VALUE | b VALUE-a VALUE | WATER SOLUBILITY |
| EXAMPLE 7 | 0.059 | 0.05 | 1.18 | 1.11 | 0.07 | TRANSPARENCY |
| COMPARATIVE EXAMPLE 6 | 0.059 | 0.05 | 1.18 | 0.90 | 0.28 | WHITE TURBIDITY |

As illustrated in Table 1 to Table 3, the results of the evaluation of water solubility were "transparency" for the tocopherol phosphoric acid ester salts of Example 1 to Example 7, in which "a value" was 1.10 or more, and excellent water solubility was shown. On the contrary, the results of the evaluation of water solubility were "white turbidity" for the tocopherol phosphoric acid ester salts of Comparative Example 1 to Comparative Example 6, in which "a value" was less than 1.10, and water solubility was not sufficient.

Even though in Example 1, "b value" was the same as that of Comparative Example 1, in Example 2, "b value"

was the same as those of Comparative Example 2 and Comparative Example 3, in Example 3, "b value" was the same as that of Comparative Example 4, in Example 4, "b value" was the same as that of Comparative Example 5, and in Example 7, "b value" was the same as that of Comparative Example 6, "a value" was larger in the Examples in any of the cases.

The reason for this is considered to be that the difference between "a value" and "b value" became small since washing was performed to remove the phosphoric acid as the by-product, before neutralizing the tocopherol phosphoric acid, in Examples 1 to 4, and Example 7.

In Example 5, since "a value" was 1.70 or less, it can be understood that the balance between water solubility and oil solubility is excellent.

## Claims

1. A tocopherol phosphoric acid ester salt,
wherein the tocopherol phosphoric acid ester salt is represented by Formula (1),

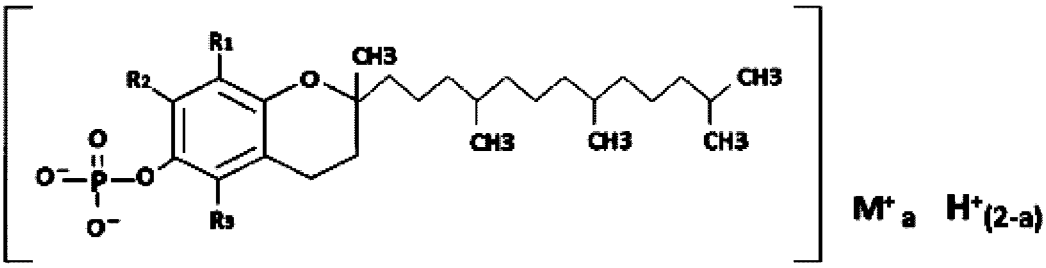

・・・(1)

in the Formula (1), "R1", "R2", and "R3" each independently represents a hydrogen atom or a methyl group; "M" represents an alkali metal; and "a" is 1.10 or more and 2.00 or less.

2. The tocopherol phosphoric acid ester salt according to Claim 1, wherein, in the Formula (1), the "a" is 1.10 or more and 1.70 or less.

3. The tocopherol phosphoric acid ester salt according to Claim 1 or 2, wherein in the Formula (1), the "M" is a sodium.

4. A skin external preparation, comprising:

    the tocopherol phosphoric acid ester salt according to any one of Claims 1 to 3.

5. A method for manufacturing the tocopherol phosphoric acid ester salt according to any one of Claims 1 to 3, the method comprising:

    generating a tocopherol phosphoric acid ester by reacting a tocopherol and a phosphorylating agent, and then, dissolving a salt precipitated along with the tocopherol phosphoric acid ester by adding a sulfuric acid aqueous solution to a reaction solution including the tocopherol phosphoric acid ester;
    washing the reaction solution after dissolving the salt to remove a phosphoric acid therefrom; and
    neutralizing the tocopherol phosphoric acid ester with an alkali metal hydroxide to generate an alkali metal salt of the tocopherol phosphoric acid ester,
    wherein an amount of the alkali metal hydroxide used in the neutralizing is controlled so that the number of moles of an alkali metal with respect to 1 mole of the tocopherol phosphoric acid ester is 1.17 to 1.88.

## INTERNATIONAL SEARCH REPORT

International application No.
PCT/JP2016/065040

A. CLASSIFICATION OF SUBJECT MATTER
*C07F9/655*(2006.01)i, *A61K8/67*(2006.01)i, *A61K31/665*(2006.01)i, *A61P17/00*(2006.01)i, *A61Q19/00*(2006.01)i

According to International Patent Classification (IPC) or to both national classification and IPC

B. FIELDS SEARCHED

Minimum documentation searched (classification system followed by classification symbols)
C07F9/00, A61K8/00, A61K31/00, A61P17/00, A61Q19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2013-523660 A (Phosphagenics Ltd.), 17 June 2013 (17.06.2013), claim 3 & US 2011/0244022 A1 claim 3 & WO 2011/120084 A1 & EP 2552486 A1 | 1,3 |
| X | JP 2007-99708 A (Nippon Menard Cosmetic Co., Ltd.), 19 April 2007 (19.04.2007), claims 2 to 3; paragraphs [0025], [0029] (Family: none) | 1,3-4 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 July 2016 (14.07.16) | 26 July 2016 (26.07.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/065040

C (Continuation). DOCUMENTS CONSIDERED TO BE RELEVANT

| Category* | Citation of document, with indication, where appropriate, of the relevant passages | Relevant to claim No. |
|---|---|---|
| X | JP 2005-53785 A (Nippon Menard Cosmetic Co., Ltd.), 03 March 2005 (03.03.2005), claims 1 to 8; examples 1 to 7 (Family: none) | 1-4 |
| X | JP 2005-53784 A (Nippon Menard Cosmetic Co., Ltd.), 03 March 2005 (03.03.2005), claims 1 to 8; examples 1 to 6, 8 to 12 (Family: none) | 1-4 |
| X | JP 2004-175712 A (Yugen Kaisha Nonogawa Shoji), 24 June 2004 (24.06.2004), paragraphs [0019], [0020], [0023] (Family: none) | 1-3 |
| X | JP 6-509351 A (LVMH Recherche), 20 October 1994 (20.10.1994), pages 5 to 6, example 1 & US 5603949 A columns 6 to 7, example 1 & WO 1993/002661 A1 & EP 597025 A1 | 1,3,5 |
| X | JP 59-44375 A (Senju Pharmaceutical Co., Ltd.), 12 March 1984 (12.03.1984), page 2, lower right column to page 3, upper left column (Family: none) | 1,3,5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015102878 A
- JP 2004026817 A
- JP 2008007428 A
- JP S5944375 B
- WO 9714705 A


### Non-patent literature cited in the description


- Pharmaceutical and Medical Device Regulatory Science Society of Japan. Yakuji Nippo Ltd. Company, 1984
- Evaluation and Registration Division of Pharmaceutical Affairs Bureau. Ministry of Health and Welfare. Yakuji Nippo Ltd. Company, 1993
- Evaluation and Registration Division of Pharmaceutical Affairs Bureau. Ministry of Health and Welfare. Yakuji Nippo Ltd. Company, 1997
- Cosmetic ingredient dictionary. Nikko Chemicals Co., Ltd,
- New cosmetic functional material. CMC Publishing, 2002, vol. 300